# EUROPEAN PATENT SPECIFICATION

(11) **EP 3 708 202 B1**
(45) Date of publication and mention of the grant of the patent: **03.12.2025**
(21) Application number: 20172466.3
(22) Date of filing: 10.10.2016
(51) Int. Cl.: A61M 5/142, A61M 5/145, A61M 5/32, A61M 5/34, A61M 5/158, A61M 5/28

(54) **AN INJECTOR NEEDLE CAP AND/OR LINER REMOVER**
ENTFERNER FÜR INJEKTORNADELKAPPE UND/ODER LINER
CAPUCHON D'AIGUILLE D'INJECTEUR ET/OU DISPOSITIF D'ENLÈVEMENT DE REVÊTEMENT

(30) Priority: 09.10.2015 US 201562284806 P; 21.01.2016 US 201662281536 P; 07.07.2016 US 201615204542; 19.09.2016 US 201615269248
(43) Date of publication of application: 16.09.2020
(62) Divisional of application: 16784692.2
(73) Proprietor: West Pharma Services IL, Ltd., 4366411 Ra'anana (IL)
(72) Inventor: Cabiri, Oz, 4526611 Hod Hasharon (IL); Hezkiahu, Ran, 4668346 Herzliya (IL)
(74) Representative: Petraz, Gilberto Luigi

(56) References cited:
- EP-A1- 2 574 355
- WO-A1-2013/058697
- US-A1- 2014 171 881

## Description

### FIELD AND BACKGROUND OF THE INVENTION

The present invention relates to a needle cap remover and, more particularly, but not exclusively, to a bifurcated needle cap remover.

U.S. Patent Application No. US 2012/0238961 discloses "a needle shield remover that reliably engages with a distal cap of an automatic injection device and with one or more needle shields coupled to a syringe of the device. When a user removes the distal cap, the needle shield remover reliably removes the needle shields (e.g., a soft needle shield and a rigid needle shield) from the syringe, thereby exposing the injection needle for performing an injection. In an exemplary assembly method, a needle shield remover is engaged to a needle shield coupled to a syringe, prior to insertion of the syringe and needle shield remover assembly into a housing of the device. This exemplary assembly method allows visual inspection, outside the housing of the device, to ensure that the needle shield remover is correctly and reliably engaged to the needle shield before the syringe and needle shield remover assembly is inserted into the housing".

U.S. Patent No. 6,843,782 discloses "a drug delivery device having a base member defining a skin-contacting surface, a syringe serving as a reservoir for the drug, and means for expelling drug from the syringe. The syringe is connected to the base member such that the longitudinal axis of the syringe is substantially parallel to the skin surface. A delivery needle is in communication with the syringe. The needle has an angled bend which directs the tip of the needle substantially perpendicular to the skin-contacting surface. In use, the tip of the needle is adapted to penetrate the skin of the subject".

International Patent Application Publication No. WO 2015/048791 discloses "a method of preparing a compound device for use. The device may include a sealed component and an active outer surface. The outer surface may be protected by a surface cover. Preparing the device may include activating the active outer surface by removing the surface cover and exposing an internal portion of the sealed component to the exterior of the device by unsealing the sealed component and synchronizing the activating and said unsealing using a coupler attached to the surface cover and the sealed component."

U.S. Patent Application No. US 2014/0171881 discloses a safety cap suitable to join a needle protector to a cover of a needle. The safety cap may have a user handle coupled thereto.

### SUMMARY OF THE INVENTION

A device for removing a needle cap, according to the present invention, is set out in claim 1. A corresponding method of assembling the needle cap remover is set out in claim 8. The dependent claims provide preferred embodiments of the invention.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRAWING

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIGs. 1A-E schematically illustrate an exemplary device for removing a needle cap having a bifurcated body, in accordance with some embodiments of the current invention, wherein *FIG. 1A* illustrates a perspective view, *FIG. 1B* illustrates a front view, *FIG. 1C* illustrates a side view, *FIG. 1D* illustrates a top view and *FIG. 1E* illustrates a perspective top view;
FIGs. 2A-C schematically illustrate an exemplary use of the cap remover in an injector device, in accordance with some embodiments of the current invention, wherein *FIG. 2A* illustrates an example of the device being used in an automatic injector assembly, *FIG. 2B* illustrates the inner cartridge and needle assembly provided in the injector of *FIG. 2A**,* and *FIG.* 2C illustrates another example of the device used in an automatic injector;

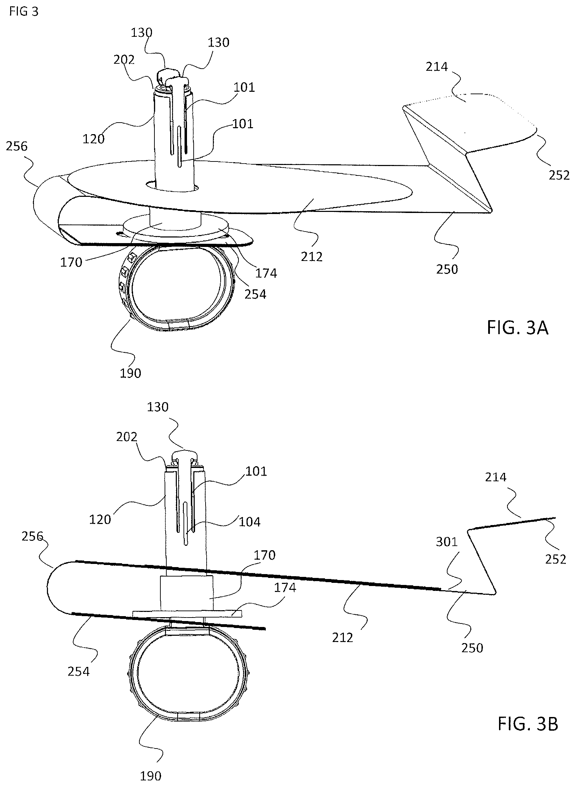

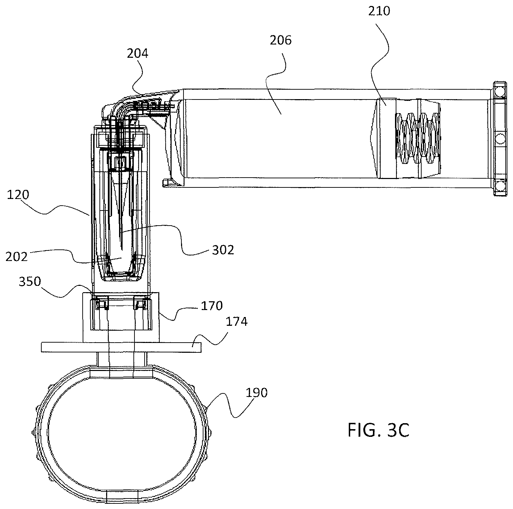

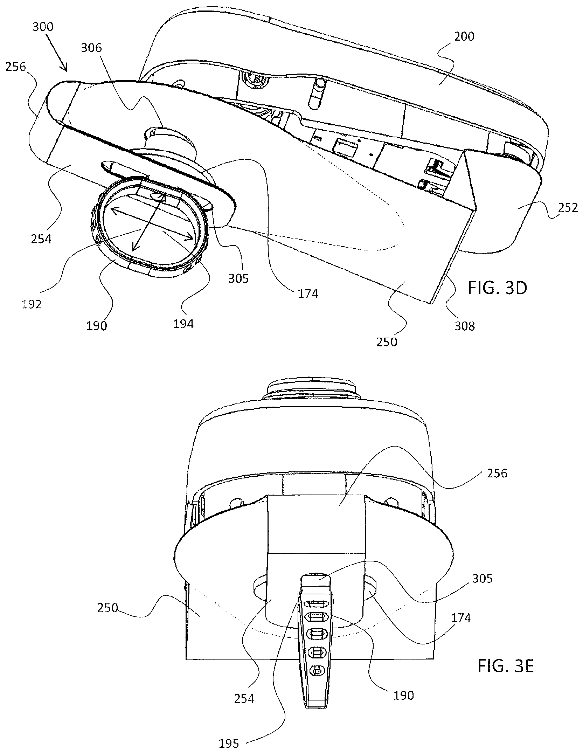

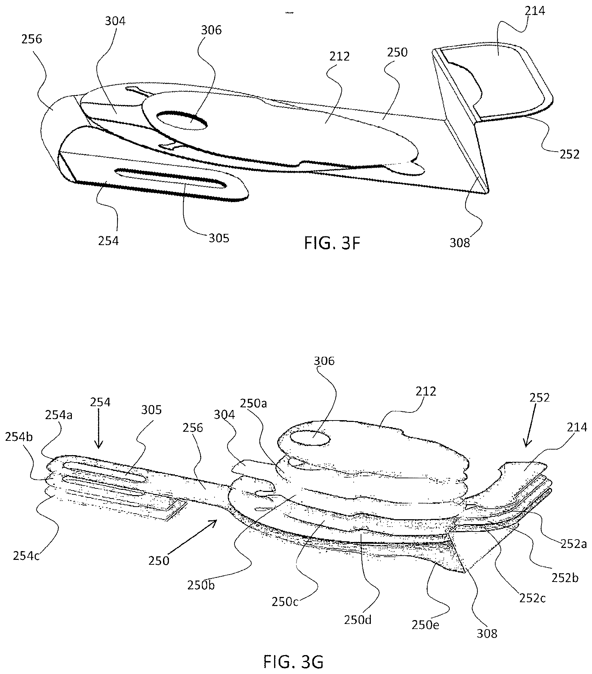

FIGs. 3A-G schematically illustrate an exemplary incorporation of an adhesive liner of an injector device into the cap remover, in accordance with some embodiments of the current invention, wherein *FIG. 3A* illustrates a perspective side view, *FIG. 3B* illustrates a side view, *FIG.* 3C illustrates a cross sectional side view of a cap remover assembled onto a cartridge, FIG. 3D illustrates a perspective view of the injector having a device cover, FIG. 3E illustrates a front view of the injector having a device cover, FIG. 3F illustrates a perspective view of the device cover and FIG. 3H illustrates an explosive view of the device cover layers, in accordance with some embodiments of the invention;

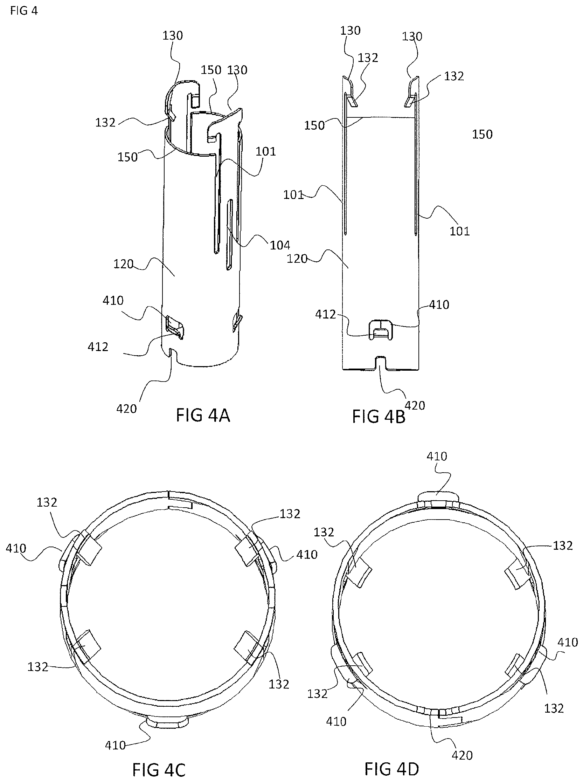

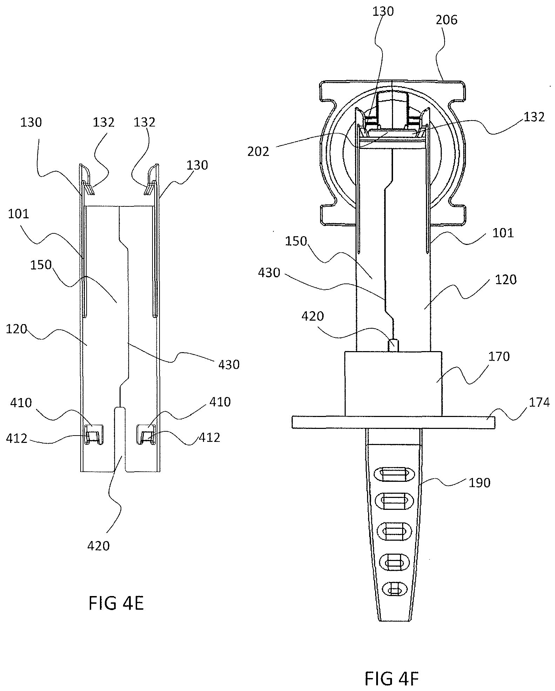

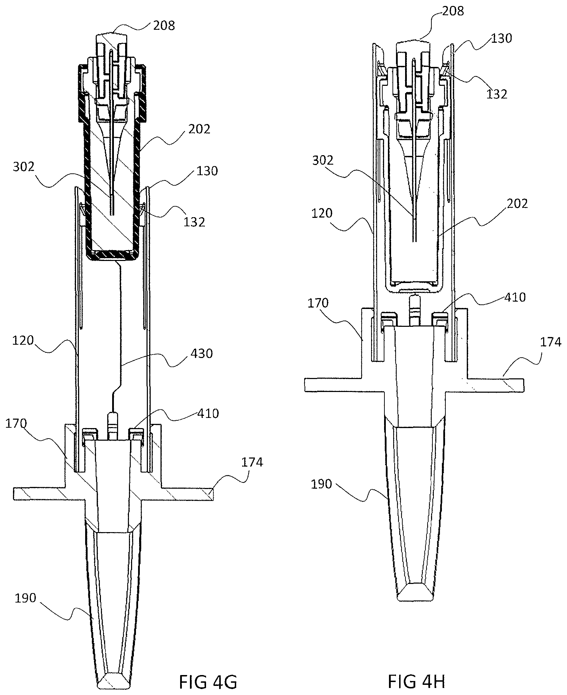

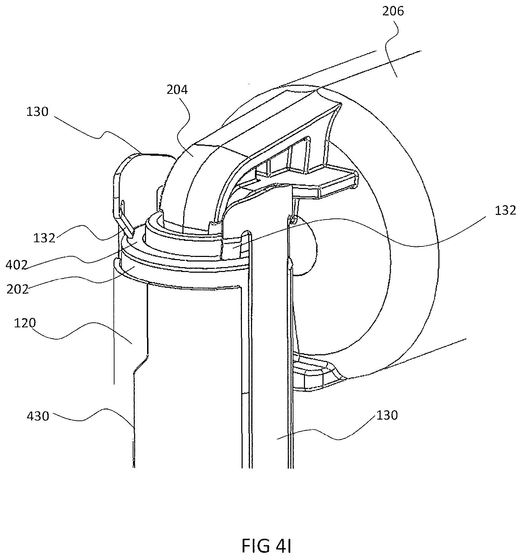

FIGs. 4A-I schematically illustrate an exemplary needle cap remover bifurcated cover and its assembly with a needle cap, in accordance with some embodiments of the current invention, wherein *FIG. 4A* illustrates a perspective view, *FIG. 4B* illustrates a front view, *FIG. 4C* illustrates a side view, *FIG. 4D* illustrates a top view, *FIG. 4E* illustrates a bottom view of a needle cap remover embodiment, and *FIG. 4E* illustrates a second needle cap remover cover embodiment, shown as part of a cap remover device assembled on a cartridge in *FIG. 4F*, and *FIG. 4G* illustrates a cross section of a front view of a needle cap remover device being pushed onto a needle cap and *FIG. 4H* illustrates a cross section of the device and the needle cap after their assembly, and *FIG. 4I* illustrates a partial perspective close up view of the top portion of the needle cap assembled with the cap remover;
FIGs. 5A-E schematically illustrate an exemplary cap remover connector having a handle, in accordance with some embodiments of the current invention, wherein *FIG. 5A* illustrates a perspective view, *FIG. 5B* illustrates a cross section view, *FIG. 5C* illustrates a top view, *FIG. 5D* illustrates a front view and *FIG. 5E* illustrates a side view;
FIG. 6 is a flow chart illustrating an exemplary process for assembling a needle cap remover onto a needle cap, in accordance with some embodiments of the current invention;
FIG. 7 is a flow chart illustrating an exemplary assembly of a needle cap remover assembly with an adhesive liner, in accordance with some embodiments of the current invention;
FIG. 8 is a flow chart illustrating an exemplary process assembling an automatic injector device, in accordance with some embodiments of the current invention;
FIGs. 9A-B schematically illustrate an exemplary multi-part cap remover, in accordance with some embodiments of the current invention, wherein *FIG. 9A* exemplifies a multi-part cap remover in an open configuration and *FIG. 9B* exemplifies a multi-part cap remover in a tight configuration;
FIG. 10 is a flow chart illustrating a process of assembling a multi-part cap remover onto a needle cap, in accordance with some embodiments of the current invention; and

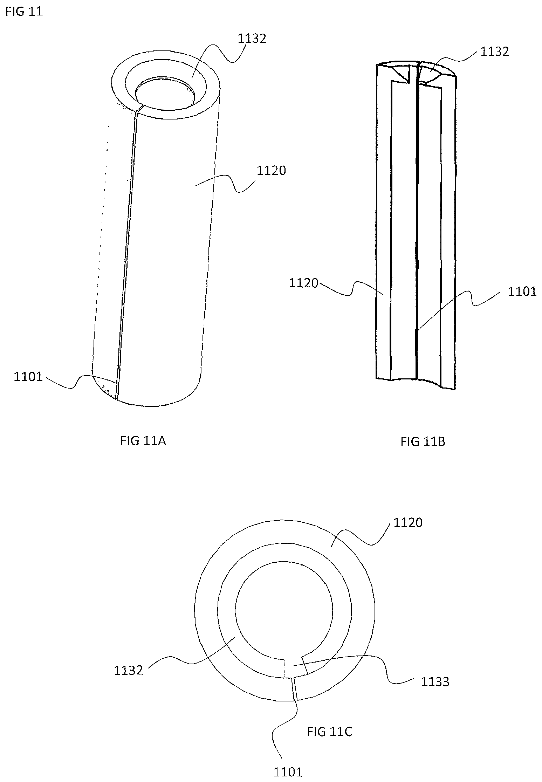

FIGs. 11A-C exemplify a singled arm needle cap remover, in accordance with some embodiments of the current invention, wherein *FIG. 11A* illustrates a perspective view of the device, *FIG. 11B* illustrates a cross-section view of the device and *FIG. 11C* illustrates a top view of the device.

### DESCRIPTION OF SPECIFIC EMBODIMENTS OF THE INVENTION

The present invention, in some embodiments thereof, relates to a needle cap remover and, more particularly, but not exclusively, to a bifurcated needle cap remover.

### Overview

An aspect of several embodiments of the invention relates to a needle cap remover configured to slide in one direction and clamp in the opposite direction. In some embodiments, the needle cap remover comprises a cover body having a longitudinal axis characterized by a distal end configured to be pushed over and/or receiving and/or enveloping a needle cap, and a proximal end configured for being pulled by a user. The body is cylindrical. The body comprises at least two longitudinal arms at least partially coupled to define a tubular structure, optionally having a diameter larger than a diameter of a needle cap.

In some embodiments, the cover body comprises at least two hooks, optionally coupled to its distal end. In some embodiments, the hooks are provided at the distal portion of the longitudinal arms. Optionally, the hooks define a diameter which is smaller than the diameter of the needle cap. In some embodiments, once the cover body is pushed over the needle cap, the hooks are deflected away from the central axis of the needle cap to allow their defined diameter to fit over the larger diameter of the needle cap. In some embodiments, deflection of the hooks is provided by elastic elements, optionally the elastic elements comprise the at least two longitudinal arms.

In some embodiments, the at least two longitudinal arms are defined by bifurcated sections in the cover body. Optionally, the bifurcated sections are configured to distribute pushing forces, potentially enabling assembly of the needle cap remover onto the needle cap without exerting too much force onto the needle cap, for example exerting no more than 100 g force, or exerting no more than 150 g force, or exerting no more than 200 g force, or exerting no more than 250 g force. Alternatively or additionally, the bifurcated sections are configured to distribute pulling forces such that potentially removing the needle cap with the needle cap remover substantially maintains an axial direction. In some embodiments, in order to pull the needle cap no more than 0.5 Kg force is applied. Alternatively, no more than 0.7 Kg force is applied. Alternatively, no more than 0.9 Kg force is applied. Alternatively, no more than 1 Kg force is applied. Alternatively, no more than 1.2 Kg force is applied. Alternatively, no more than 1.5 Kg force is applied. In some embodiments, the cap remover cover body has an integral proximal end and a bifurcated distal end.

In some embodiments, the bifurcated end of the cap remover comprises 4 slits which divide the bifurcated end into 4 longitudinal arms. Alternatively or additionally, the bifurcated end of the cap remover comprises 5, or 6, or 7, or 8 slits. Alternatively or additionally, the bifurcated end of the cap remover comprises 2, or 3 slits. Optionally, the slits are offset with respect to the longitudinal axis of the cover body, for example, tilted by an angle range of 1°-20°. Alternatively, at least some of the slits are tilted by an angle range of 15°-35°. Alternatively at least some of the slits are tilted by an angle range of 30°-45°, or any range smaller, larger or intermediate. Optionally, the slits define a triangular portion.

In some embodiments, the bifurcations are arranged symmetrically around a perimeter of the cap remover. Optionally, the bifurcations are arranged equidistantly. Alternatively, the bifurcations are arranged such that at least two different sizes of bifurcated portions, optionally in the form of elongated arms, are provided. Optionally, pairs of equally sized bifurcated portions are arranged substantially diametrically.

In some embodiments, bifurcation portions are geometrically distributed in a configuration requiring a relatively small force when pushing the cap remover onto a needle cap and a relatively large force when pulling the cap with the cap remover. Potentially, the relative location of the slits affects the directionality of the forces which are exerted on the cap remover. Alternatively or additionally, the elasticity of at least a portion of the bifurcated portions affects the directionality of the forces exerted on the cover body. In some embodiments, elasticity of the cap remover portions is affected by the perimeter length taken up by the portions. Alternatively or additionally, elasticity of the cap remover portions is affected by the composition of the portions material.

In some embodiments, the distal portion of the longitudinal arms is coupled to at least two hooks, and/or fingers. Optionally, the hooks cause the perimeter of the cap remover to be smaller than the perimeter of the needle cap. In some embodiments, when the cover body is pushed over the needle cap, once the hooks pass the top sill of the needle cap, the smaller perimeter allows the hooks to overhang the top sill. In some embodiments, at least some of the bifurcated portions, optionally in the form of longitudinal arms, are elastic enough to deflect away from the longitudinal axis of the cap remover at least to an extent which allows the smaller perimeter defined by the hooks to fit over the larger perimeter of the needle cap.

Optionally, the hooks operate at a snap-fit mechanism. In some embodiments, at least some of the hooks include projections extending towards a central axis of the cap remover. In some embodiments, when the cap remover is pushed over the needle cap, once the hooks pass over the top sill of the needle cap, the hooks and/or elastic arms bounce back and/or snap towards the central longitudinal axis. In some embodiments, when a pulling force is applied to a cover body assembled onto a needle cap, the hooks are configured to exert force in an axial direction towards to the bottom portion of the needle cap. Optionally, hooks in the form of inward projections include projections which are tilted towards the bottom portion of the needle cap, potentially creating a resisting clasp when the cover body is pulled and the projections are pushed against the top sill of the needle cap. Alternatively or additionally, snap projections are perpendicular to the longitudinal axis of the cover body.

In some embodiments, the position of the slits affects the resistance exerted by the snaps which are being pushed on to the needle cap, optionally leading to the pulling of the needle cap with the cap remover when pulling the cap remover. Alternatively or additionally, the size of the slits affects the snaps being held. In some embodiments, the position configuration of the slits allow the elastic portions to deflect away from the central axis of the cap remover when pushed over the needle cap in one direction, but at the same time cause the snaps to hold onto the needle cap when the cap remover is pulled in the opposite direction, and prevent the elastic portions from deflecting. In some embodiments, intermediate slits are provided, optionally in the elastic portions, optionally positioned in proximity to the middle portion of the longitudinal axis of the cap remover. In some embodiments, intermediate slits do not reach the edge of the cap remover body.

In some embodiments, the bifurcated portions are optionally symmetrically arranged around the perimeter of the cap remover. Alternatively or additionally, the bifurcated portions are symmetrically arranged with respect to a central axis. In some embodiments, four slits are provided, partitioning the cap remover body distal end into four portions, optionally two elastic portions and two rigid portions. Elastic portions are for example arms which can deflect away from the central axis of the cover body to at least allow coupled hooks to be fit over the perimeter of the needle cap. Rigid portions are for example portions of the cover body which are not configured to deflect away from a central axis of the cover body. In some embodiments, elastic portions are positioned substantially diametrically. Alternatively or additionally, rigid portions are positioned substantially diametrically.

In some embodiments, the elastic portions take up a smaller perimeter length with respect to the rigid portions. For example, the elastic portions comprise 10-40% of the body cover perimeter, while the rigid portions comprise 90-60% of the body cover perimeter. Alternatively, the elastic portions comprise 20-30% of the body cover perimeter, while the rigid portions comprise 80-70% of the body cover perimeter. It is a potential advantage to provide larger rigid portions and smaller elastic portions, as it contributes to the rigid and elastic characteristics of the portions, respectively.

In some embodiments, a connector, for example a body containing a clamping mechanism, is provided to be assembled onto the cap remover cover body, optionally to connect the cover body to other features. Alternatively or additionally, the connector acts as a securing mechanism and is optionally shaped and sized to add mechanical stability to the cover body. In some embodiments, the cover body is positioned within an injector system and the connector can be operated from the outside of the injector system. In some embodiments, the cap remover comprises fasteners configured to interlock with the connector. Potentially, the fasteners prevent lateral movement of the cover body with respect to the needle cap. Optionally, the connector further comprises a user handle. In some embodiments, the fasteners interlocking with the connector limit force exertion in a rotational direction, potentially precluding sliding of the needle remover body around the needle cap and/or preserving an orientation of the needle cap remover with respect to the needle cap.

An aspect of several embodiments of the invention relates to a medicament delivery system, for example an autoinjector comprising a needle cap remover. In some embodiments, a needle is installed into an autoinjector and is covered with a needle cap. Subsequently, an adhesive liner is installed to the injector. In some embodiments, removal of the needle cap also brings about removal of the adhesive liner. In some embodiments, the needle cap remover is positioned perpendicularly to the base of the injector system, to allow a user a more intuitive pulling direction.

An aspect of several embodiments of the invention relates to a needle cap remover having at least two separate fastening mechanisms. In some embodiments, a first fastening mechanism comprises at least two enveloping arms sized and shaped to envelop at least a portion of a needle cap. In some embodiments, the arms are coupled in at least a portion across their longitudinal axis, optionally enabling the arms to hinge-tilt from the coupled position. Optionally, when the distal portions of the arms tilted away from the longitudinal axis of the cap remover, the arms define an open configuration, and when the arms are not tilted away they define a closed enveloping configuration. In some embodiments, a second fastening mechanism is configured to latch the arms in their closed configuration, optionally in the form of a connector which may also connect arms being inside an injector system to an outside of the injector system.

In some embodiments, a closed configuration is defined by having the members enclosed around the needle cap. An opened configuration is defined by having the members spaced to have a diameter greater than the needle cap, optionally, at least as wide as the widest diameter of the needle cap. In some embodiments, the arms are provided with fingers that are configured to overhang a top sill of the needle cap once the arms are in their closed configurations. Optionally, when the cap remover is being pulled, the connector keeps the arms in their closed configuration and the fingers push down on the cap and remove it from the needle, optionally in substantially a downwards axial direction.

An aspect of some embodiments of the invention relates to a device protective cover allowing access of a needle cap remover into an inner portion of the device, and designed to be assembled onto the needle cap remover and its user pulling handle after the remover is assembled with its user handle. In some embodiments, the protective cover comprises a plurality of layers, optionally at least an adhesive layer and a liner layer protecting the adhesive surface of the adhesive layer. In some embodiments, protective cover is designed to be removed by pulling the needle cap remover through its user handle in a linear direction. Optionally, only a portion of the protective cover area is attached to the injector, while an extending edge extends from the end of the portion attached. In some embodiments, the extended edge is folded and/or bent to be oriented facing the attached portion, optionally by handing the extended edge onto the user handle.

In some embodiments, the protective cover comprises at least two apertures. Optionally, a first aperture allows insertion of the needle cap remover into the injector inner portion. In some embodiments, the first aperture is sized to match the circumferential shape of the needle cap remover, optionally being wider than the needle cap remover. It is a potential advantage to keep the first aperture small enough to allow sufficient stability when the device is adhered to the patient and the needle is penetrating through this aperture. It is another potential advantage to design the aperture to have a width smaller than a human finger, to prevent a user from inserting his figure into the needle region, e.g. smaller than 15 mm, and/or smaller than 10 mm, and/or smaller than 5 mm. In some embodiments, the cap remover body has no outward protrusions in the region which needs to be inserted into the aperture, in order to preserve the option for a small aperture width as allowed by the body's own width.

Also optionally, a second aperture allows fitting over the user handle. In some embodiments, a user handle is provided as having a long dimension and a short dimension. Optionally, the second aperture is sized as an elongated slit, having a length being longer than the long dimension and shorter than the short dimension of the handle. In some embodiments, the elongated aperture is assembled onto the handle by first orienting the aperture to face the shorter dimension and then rotating the edge of the cover such that the elongated aperture slides towards the longer dimension, optionally eventually being oriented to face the long dimension. In some embodiments, when placing the liner over the handle, no force is applied to the cap remover and/or needle cap. Alternatively, a force smaller than 50 g, and/or 100 g, and/or 150 g is applied.

In some embodiments, the edge is stabilized in place by the handle becoming wider as the aperture slides toward the long dimension and by constraining the edge of the aperture against the wide portion of the handle, being wider than the aperture. Alternatively, the edge is directed to pass the widest portion of the handle and reach a narrower portion, causing the aperture edge to overhang over the wide portion serving as a shoulder portion. Alternatively or additionally, the handle is rotated after the edge is passed over it, such that the long dimension of the handle is found at an angle to the length of the aperture, thereby preventing a slide of the edge in the direction it passed over. Alternatively or additionally, the edge slides to an offset direction from the center of the long dimension, misaligning the aperture with the handle's long dimension.

In some embodiments, at least a portion of an edge of at least one of the apertures also comprises a supporting layer in the form of a rigid layer, rigid enough to provide mechanical stability to prevent bending at the forces which are applied when pulling the cover. Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary embodiments

### 1 An exemplary needle cap remover

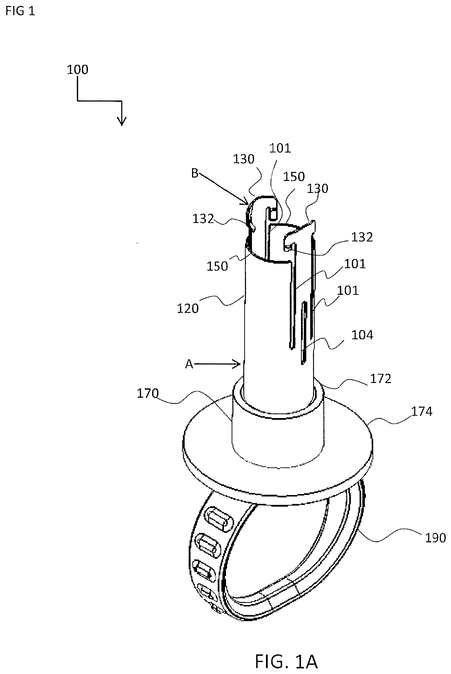

Referring now to the drawings, *Figs. 1A-E* illustrate a needle cap remover **100** designed to remove a needle cover in accordance with an embodiment of the current invention. In some embodiments, needle cap remover **100** is designed such that forces exerted on the needle cover do not disturb a sterility state of a needle cap enveloping a sterile needle.

In some embodiments, needle cap remover **100** has an elongated cover body **120** configured for at least partially covering a needle cap. Elongated body **120** in some embodiments is sized and shaped to envelope a needle cap, optionally tightly. Elongated body **120** optionally includes a bore **124**. For example, body **120** and/or bore **124** are cylindrical. Alternatively, a portion of body **120** and/or bore **124** may be conical for example being tapered and/or having the shape of a conical section and/or funnel shaped. Body **120** has a distal end **B** which in some embodiments is found in proximity to a top portion of a needle cap, i.e. the portion having the needle receiving bore. Body **120** has a proximal end A, which in some embodiments is found in proximity to a bottom portion of a needle cap, i.e. the portion closest to the needle tip.

In some embodiments, elongated body **120** comprises at least two bifurcations, optionally dividing body **120** into at least two portions, potentially portion **130** and/or portion **150**. In some embodiments, at least a first **130** of the at least two portions has elastic properties, optionally, the two portions are in the form of longitudinal arms. Alternatively or additionally, at least a second **150** of the at least two portions has rigid properties, e.g. is more rigid than first **130** portion. In some embodiments, elastic portions **130** are elastic enough to deflect away from a central axis of the cover body by an angle having a range of 1°-5°. Alternatively, elasticity is enough to enable deflection by an angle having a range of 10°-15°. Alternatively, elasticity is enough to enable deflection by an angle having a range of 15°-20°. In some embodiments, reaching an angle greater than the elasticity range results in plastic deformation of the arms, optionally, reducing the resilient snapping of the arms to their original position. Alternatively, deflection beyond the higher threshold of the range results in breakage of the arms.

In some embodiments, elasticity and/or rigidity is affected by the size, shape and/or position of slits **101** created by the bifurcations, optionally their longitudinal length and/or position along the longitudinal axis of body **120**. Alternatively or additionally, the width of slits **101** influences the elasticity of its adjacent portions. An optional range for the longitudinal length of slits **101** is between about 5 mm and about 20 mm. Alternatively, a length of slits **101** is between 7 mm and 10 mm. An optional range for the width of slits **101** is between 0.3 mm and 3 mm. Optionally, the width of slits **101** is 2 mm.

Alternatively or additionally, elasticity and/or rigidity are determined by the position of slits **101** around the perimeter of body **120** and thus according to the perimeter length taken up by elastic portion **130** and/or the perimeter length taken up by rigid portion **150**, which are defined by the positions of slits **101**. In some embodiments, slits **101** define the partition of the distal portion of body **120** into relatively elastic **130** and relatively rigid **150** portions. Optionally, portions **130** are elastic relative to portions **150**, and portions **150** are rigid relative to portions **130**. In some embodiments, elastic portions take up to 50% of the perimeter of body **120**. Alternatively, elastic portions take up to 40 % of the perimeter of body **120**. Alternatively, elastic portions take up to 30 % of the perimeter of body **120**. Alternatively, elastic portions take up to 20 % of the perimeter of body **120**. Alternatively, elastic portions take up to 10 % of the perimeter of body **120**.

In some embodiments, at least one intermediate slit **104** is provided, optionally in elastic portion 130, potentially increasing its elasticity. Optionally, intermediate slit **104** is surrounded by body **120** and does not continue all the way to the edge of **120**. In some embodiments, intermediate slit **104** is positioned centrally between two of slits **101**. Alternatively or additionally, intermediate slit **104** is positioned asymmetrically with respect to slits **101**, potentially leading to a favorable direction in reaction to force exertion on device **100.** Alternatively or additionally, intermediate slit **104** is positioned in proximity to only one of slits **101**. Optionally, intermediate slit **104** has identical dimensions to slits **101**. Alternatively, intermediate slit has smaller or larger length. Alternatively or additionally, intermediate slit has smaller or larger width.

In some embodiments, elastic portion **130** and rigid portion **150** have distinct lengths. Optionally, elastic portion **130** extends beyond rigid portion **150** towards the distal end of device **100.** In some embodiments, at least some of the elastic portions comprise snaps **132** at their distal end. Snaps **132** are configured in some embodiments to snap body **120** onto the needle cap's top portion. Optionally, snaps **132** create a perimeter which is smaller than the perimeter of the needle cap. In some embodiments, due to the elasticity of elastic portions **130**, when device **100** is pushed onto the needle cap, elastic portions **130** are directed away from the central axis of body **120**. Optionally, once snaps **132** pass the top portion of a needle cap, they snap into their original position, essentially clasping the needle cover **100** over the needle cap and/or clasping over the top of the needle cover and/or over a top sill of the needle cover. Optionally snaps **132** are angled inward and/or wedged into the top and/or a top sill of the needle cap, being tilted with respect to the central longitudinal axis of the needle cap. In some embodiments, the snaps are deflected towards the central longitudinal axis of the needle cap. Optionally the angle of snaps **132** pulls the distal portion of elastic portion **130** inward (e.g. towards the central axis of the cap) when the cap remover is used to pull the cap.

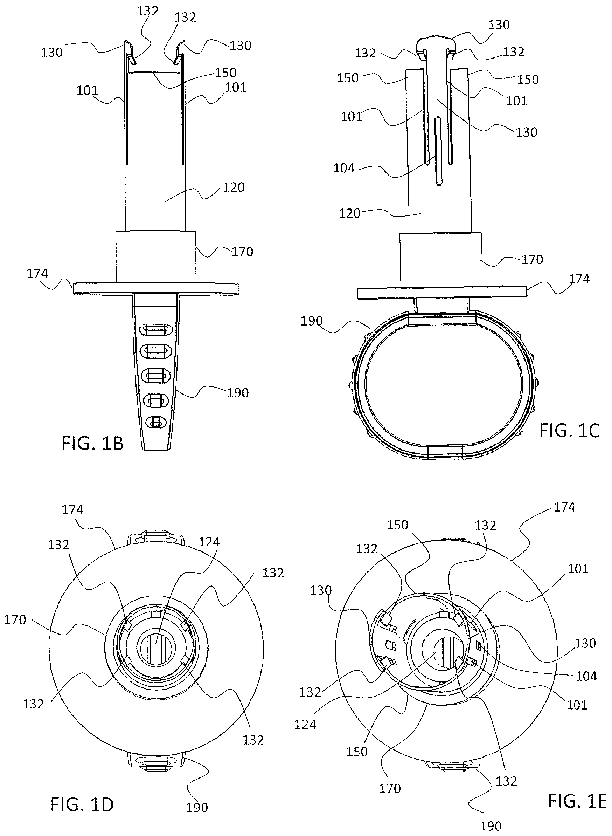

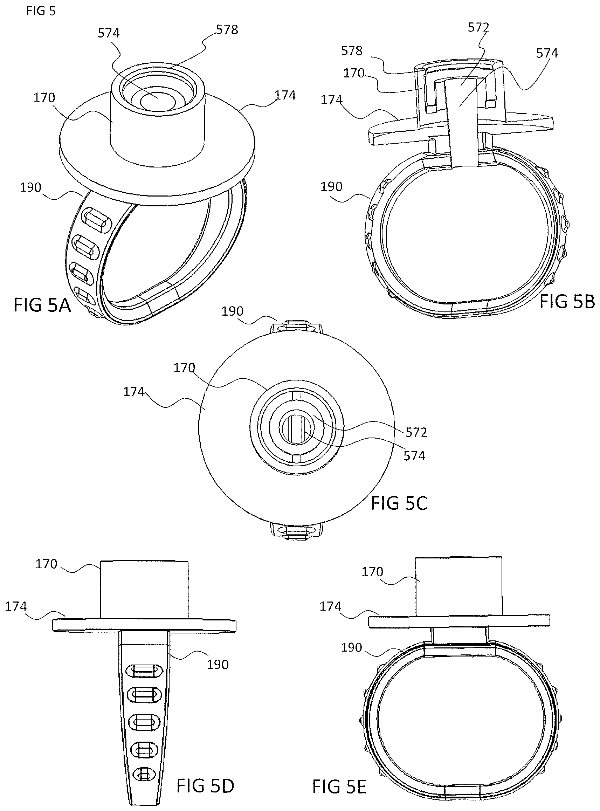

In some embodiments, needle cover **100** further comprises a user handle **190**, optionally in the form of a ring as illustrated in *FIG. 1**.* Optionally, user handle **190** is interconnected to cover body **120** by a connector, optionally having connector body **170** for connecting to cover **120** and connector base **174** potentially enabling easier gripping of cap remover **100**. In some embodiments, connector body is a separate feature which is connected to body **120**, optionally through connector ring **172**, further optionally including fastening means **410** configured to interlock ring **172** and body **120**, for example as shown in *FIGs 4* *and* *5**.* Alternatively, connector base **170** is provided as a continuation of body **120**, without being a separate feature. Optionally, fasteners **410** are configured to interlock the body cover with the connector such that a lateral movement of the cover body **120** with respect to its enveloped needle cap is inhibited.

In some embodiments, cover body **120** comprises a recess **420**, optionally configured to complement protruding longitudinal guides in the needle cap, thereby guiding the direction of inserting the cover body over the needle cap. In some embodiments, at least 2 recesses **420** are provided. Alternatively, at least 3 recesses are provided. In some embodiments, recesses are provided in the proximal portion of the cover body.

Optionally, connector base **170** and/or body **120** comprise complementary longitudinal sections, which optionally facilitate orientation of the connector base **170** with respect to body **120** when assembling the two together. Longitudinal sections optionally include grooves. Alternatively or additionally, longitudinal sections include protrusions. In some embodiments, an inner portion of body **120** comprises complementary longitudinal sections configured to complement sections provided in a needle cap, potentially aiding in a correct orientation of body **120** with respect to the needle cap. Also potentially, complementary sections can assist in reducing the force needed to assemble cover **120** onto a needle cap.

### 2 Exemplary usage of the needle cap remover in an exemplary system of a drug delivery device

In some embodiments, a needle cap remover is used with a drug delivery injector system. Optionally, the injector is provided to the user after the system has been preassembled with a medicament, contained in a cartridge having a needle. Optionally the needle is embedded within a housing of an injector system and/or not visible to the user. It is potentially desirable that the medicament and the needle are preserved in sterility until they are used or just before use. For example it is potentially beneficial, to provide the device to the user while the needle is still protected by the needle cap. Optionally the needle cap is internally embedded within the housing. A needle cap remover and/or a handle as provided in accordance with some embodiments of the invention is assembled onto the needle cap, potentially having an internal portion residing inside the housing, while having an external portion outside the housing and being available for a user to pull.

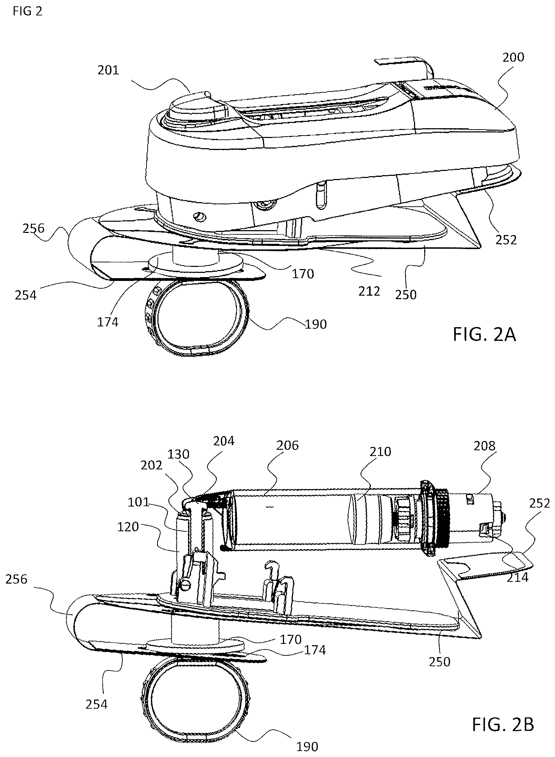

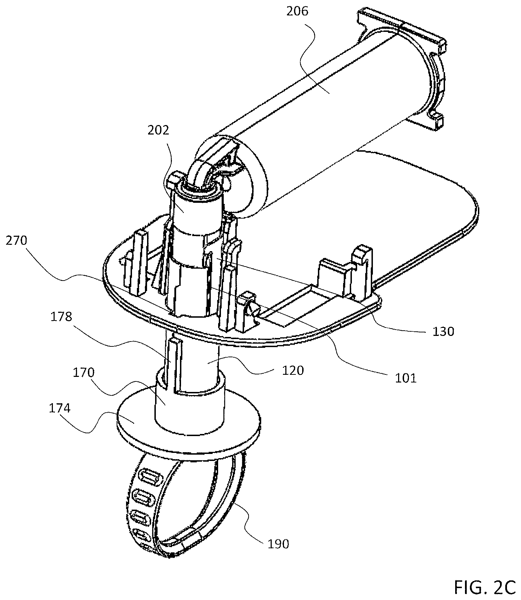

Reference is now made to *Fig. 2* illustrating an exemplary drug delivery device in the form of an automatic injector, being in a secure state for example safe for transport, and having cap remover **100** at least partially embedded. In some embodiments, adhesive liner **250** covers the activation zone of the device and/or an adhesive. Optionally, adhesive liner **250** is associated with cap remover **100** such that pulling cap remover **100** also leads to peeling of adhesive liner **250**. In some embodiments, cap remover **100** serves as a coupler, optionally synchronizing removal of the needle cap and unsealing of the activation zone of the device, optionally by peeling a protective cover layer.

In some embodiments, an adhesive section of an injector system is provided having two sections, a main adhesive **212** and a secondary adhesive **214**, optionally adhesive **214** having a smaller contact area than adhesive **212**. A potential advantage of providing at least two adhesive sections **212** and **214** is a guided removal of the device from the user's body, as an adhesive having a smaller contact area is likely to detach first.

In some embodiments, cap remover **100** and/or its associated adhesive liner **250** are associated with a battery isolator. Optionally, once cap remover **100** is pulled and/or adhesive liner **250** is peeled, battery isolator is also removed allowing to power on the injector system. In some embodiments, once power is on, the injector can no longer be turned off and/or repacked in its original state. Alternatively or additionally, once power is turned on, a mobile device is alerted. Optionally, a bar code is provided in the injector system for associating a mobile device to communicate with the injector system.

*FIG. 2A* illustrates an exemplary injector system having an injector housing **200** and operation button **201**, and having cap remover device **100**, optionally in proximity to the operation button **201**. In accordance with some embodiments, the injector system comprises a two sectional adhesive **214** and **212**, optionally covered by adhesive liner **250**. In some embodiments, adhesive liner **250** comprises a top portion **252** for covering sections **212** and **214**, optionally adhesive **212** and adhesive **250** comprise an aperture for allowing access to the needle and/or needle cap and/or needle cap remover from outside housing **200**. In some embodiments, adhesive liner **250** also comprises a bottom portion **254**, optionally connected to top portion **252** through bending **256.** In some embodiments, bottom portion **254** is associated with needle cap remover **100**, optionally by having an aperture for allowing device **100** to extend from within housing **200**, through top portion **252** and through bottom portion **254**. Optionally, bottom portion **254** is positioned between handle **190** and connector base **174**. In some embodiments, connector base **174** comprises a plate, optionally having a diameter larger than bore **124** of body **120.** Potentially, once handle **190** is pulled, connector base **174** pulls adhesive liner bottom portion **254**, which through bend **256** leads to peeling top cover **252** starting from the device extreme end being closest to bend **256**. In some embodiments, connector **170** comprises an orientation feature, such as for example beam **178,** optionally sized and shaped to fit into housing **200**, for example at slit **270**, as shown in *FIG. 2C*. Potentially, orientation feature **178** is configured to guide the insertion of cap remover device in a specific rotational orientation. Optionally, at least one fastener **350** is provided in connector **170** and/or cover body **120,** potentially setting the orientation of connector **170** with body **120.**

*Fig. 2B* illustrates an embodiment of an injector system having a needle cap **202** associated with a cannula **204** of a medicament cartridge **206,** resting on a frame of housing **200** of the injector, optionally the injector system is operated automatically by means of motor **208**, potentially pushing plunger **210.** Putting pressure on the proximal end of the injector (which is optionally covered by front cover **250**) optionally pushes needle protector **214** against the frame. Optionally putting pressure on the proximal end of the injector will not activate the injector and/or will not un-shield the needle. Needle cap **202** optionally acts at a physical shield covering the tip of a needle associated with cannula **204**.

Optionally, needle cap **202** includes a rigid outer shell and a sterile rubber core. When needle cover **202** is installed over a sterilized needle, the rubber core may protect the sterility of the needle and/or the shell may protect the needle from causing a stick hazard. Alternatively or additionally, a needle is covered with a single cover (either rubber or rigid) that preserves sterility and/or prevents a stick hazard.

For example, a prefilled syringe may be installed into an autoinjector with a needle already covered with needle cover **202**. Before shipping the injector, adhesive liner **250** may be installed onto the injector. In some embodiments, a needle cap remover **100** is pushed over cap **202**, optionally additionally serving as a needle cap protector. Cap remover **100** in some embodiments includes clasps (for example snaps **132**) which engage cap **202.** When needle cap remover **100** is removed from the injector, clasps **132** may pull off needle cap **202**.

### 3 A needle cap remover including an adhesive liner

Reference is now made to *FIG.* 3, illustrating perspective and side views of a needle cap remover optionally having a cover **120**, and/or adhesive liner **250** and/or handle **190.**

*Figs 3A and 3B* illustrate an injector needle cap remover and adhesive liner in accordance with an exemplary embodiment of the present invention. The cap remover includes for example a handle **190** and/or a needle cap cover **120** and/or an adhesive liner **250** for covering an adhesive **212/214** and/or for covering an activation zone of the injector system. Handle **190** and/or cover **120** and/or cover **250** are optionally connected to a battery insulator. Removing the cover optionally places the injector into an enabled state.

In some embodiments needle cap remover **100** serves as a peeler for an adhesive liner **250.** For example, cap remover **100** is optionally hinged and/or flexible. Optionally, handle **190** is located at the center of cap remover **100.** In some embodiments, handle **190** does not pull an adhesive liner **250** away from the whole surface of the adhesive **214/212** all at once (an act that would potentially require a large force to overcome the sticking force over a large surface). When handle **190** is pulled, the center portion of cap remover **100** optionally moves away together with adhesive liner bottom portion **254.** For example cover **254** is optionally is pulled away in such a way as to peel adhesive liner bend **256** following by peeling of top portion **254** bit by bit. Peeling is optionally from an extreme edge of main adhesive **212** and/or optionally peeled towards the opposite end, finally removing top portion **252** from secondary adhesive **214.**

In some embodiments, adhesive **212** is provided onto a hinged plate **301.** Optionally, once needle cap remover **100** and cover **250** are removed, a user can attach the device onto an injection site and optionally collapse hinged plate **301** to be parallel to the device. In some embodiments, a sensor senses the orientation of the hinged plate **301** with respect to the device, only allowing operation when the two are aligned. Alternatively or additionally, once plate **301** is parallel, a mechanical mechanism allows for operating the injection, such as for example, by tilting a blocking feature, such as an internal plate.

Reference is now made to *FIG. 3C*, illustrating a cross section of a side view of a cap remover device assembled onto a needle cap shielding a needle of an injector system, in accordance with some embodiments of the invention. In some embodiments, cover body **120** is configured to be pushed over needle cap **202** which shields a sterility state of needle **302.** Optionally, pushing body **120** over cap **202** does not disturb the sterility state of the needle. In some embodiments, cover body **120** comprises a cylindrical tubular shape. Alternatively, only a portion of cover body **120** is defined as tubular, and this portion potentially couples at least two arms provided by the cover body **120.** Alternatively, cover body is bifurcated to define at least two arms, optionally elastic.

In some embodiments, the elastic portions of cover body **120** allow it to be pushed over cap **202** in spite of having elements defining a smaller perimeter than the largest perimeter of cap **202.**

Reference is now made to *FIGs. 3D-3G*, illustrating exemplary embodiments of a device cover, optionally protecting adhesive properties which are possibly used to couple the device base to a patient's body.

In some embodiments, housing **200** includes in its base a device cover **300**. In some embodiments, cover **300** comprises at least two layers, one being an adhesive layer and the other being a liner for protecting the adhesive surface of the adhesive layer. In some embodiments, an adhesive surface of cover **300** assist a user to hold an injector steady on the skin of a patient for an extended period. Optionally, the adhesive surface is intermittent throughout the surface of cover **300.** In some embodiments, cover **300** is partitioned at point **308**, dividing the adhesive surfaces of the device between main section **250** and secondary section **252**, optionally section **252** having a smaller surface area, potentially leading a user in the direction the device is removed, due to the ease of extracting the smaller adhered area with respect to the larger adhered area. In some embodiments, folding consists of bending and/or arching, and/or curving and/or winding and/or twisting. Optionally, section **250** and/or section **254** have circular shapes. Alternatively or additionally they may have a shape other than circular, for example oval, rectangular and/or irregular.

In some embodiments, cover **300** comprises at least one portion for connecting to a base of housing **200**. In some embodiments, cover **300** comprises an extension 254 extending beyond a surface area of the housing base. Optionally, extension 254 is folded over at section **256** to be positioned to face portion **250** of cover **300**.

In some embodiments, cover **300** comprises at least two apertures **305** and **306**. In some embodiments, a first aperture **306** comprises in section **250** is sized and shaped to allow insertion of needle cap remover body **120** through it and into the inner portion of the injector device, which comprises the needle. Optionally, aperture **306** extends through the adhesive liner and the protective liner, and any other layer comprised the protective cover **300**. In some embodiments, aperture **306** comprises a circumference shape matching a circumference shape of the needle cap remover body **120** and optionally, having a width being no more than about 1 mm, or about 0.7 mm, or about 0.5 mm, or about 0.3 mm wider than the width of the remover body **120**.

Optionally, the adhesive liner is folded over at point **256**, forming an extension **254** which directs a linear unsealing force into a guided peeling force starting from the folded edge. In some embodiments, a second aperture is provided in extension 254 of cover **300** which extends from the cover portions being connected to the device base, such as section **250** and/or **252**, through bent portion **256**. Optionally, the second aperture **305** is used to assemble section **254** onto the connector of needle cap remover body **120**. In some embodiments, needle cap remover device serves as a coupler, coupling the needle cap at its distal side and the user handle **190** at its proximal side, optionally through connector **170**. In some embodiments, the user handle comprises a protruding element opposite said coupler, e.g. being proximal to the proximal end of the needle cap remover **100**.

In some embodiments, second aperture **305** comprises an elongated orifice. In some embodiments, the elongated orifice is sized to be pulled over a user handle **190** extending from needle cap remover body **120**, optionally connected to it through connector **170**. Optionally, user handle **190** comprises a protruding element having a long dimension **194** and a short dimension **192**. In some embodiments, elongated orifice **305** has a length which is longer than short dimension **192** and longer than long dimension **194.** In some embodiments, user handle **190** has a widening portion towards its connection with the connector **170**, optionally ending in shoulder **195**, shown in *FIG. 3E**.* **In** some embodiments, a width of orifice **305** is shorter than a width defined by shoulder **195.** In some embodiments, orifice **305** is at least partially made of a material which is elastic and/or resilient enough to allow pushing the orifice over the shoulder, despite its shorter width. Optionally, extension 254 is held over handle **190** by overhanging shoulder **195.** Alternatively or additionally, shoulders **195** include the widest portion of the narrow portion of handle **190**, sized to be not smaller than a width of orifice **305**. Optionally, orifice **305** is stabilized onto shoulders by friction.

In some embodiments, an additional supporting layer is engaged with the protective cover, optionally being intermittent and not continuous over an area of cover **300**. In some embodiments, supporting layer is made of a material being rigid enough to increase a rigidity of the protective liner, potentially providing mechanical strength for cover **300**. Optionally, supporting layer is provided at least in the sections comprising apertures **305** and **306**. In some embodiments, supporting layer is not found in extension **256**.

In some embodiments, an injector housing is provided with cover **300** being at least partially connected to its base, optionally by an adhesive layer. Optionally, the injector is assembled by interlocking a cartridge having a capped needle within an inner portion of housing **200**, such that the cartridge central axis is perpendicular to the cover, and the capped needle is centrally aligned with aperture **306**. In some embodiments, a distal end of needle cap remover is inserted into the inner portion of the base of housing **200** through aperture **306,** and is pushed over the capped needle. Optionally, the proximal end of cover body **120** remains protruding externally to housing **200**.

In some embodiments, a user handle is connected to the proximal portion of body **120**, optionally through connector **170**. In some embodiments, user handle **190** is characterized by a protruding element extending outwardly from the base and having a short dimension **194** and a long dimension **192**. In some embodiments, extension **254** is folded over the user handle **190** by first orienting aperture **305** to face the shorter dimension **194** of handle **190**, followed by rotating the cover extension **254** over the handle until the aperture **305** is oriented to face the longer dimension **192.**

In some embodiments, aperture **306** and **305** are centrally aligned. Alternatively or additionally, aperture **305** slides across along the long dimension **192** of handle **190**, such that a longitudinal axis is not centrally aligned with aperture **306** and/or with handle **190**. Potentially, sliding aperture **305** with respect to handle **190** further stabilizes its position and prevents rotation of the aperture **305** in the reverse direction. Optionally, slide of aperture **305** with respect to handle **190** is produced with extension **256** is stretched between section **250** and extension **254.**

In some embodiments, handle **190** comprises a widening portion extending in a direction from a proximal end defined as being away from housing **200** and widening when approaching a distal end of the handle defined as being proximal to housing **200**, optionally being widest as shoulder portion **195.** In some embodiments, aperture **305** is passed over the widening portion and is then constrained against the widened portion, potentially being held in place by friction forces. Alternatively, aperture **305** is passed over the widening portion a narrower portion being distal to the shoulder 195, and potentially the extension **254** is stabilized in place by overhanging an edge of aperture **305** over the widening portion, and/or shoulder **195**.

In some embodiments, needle cap remover **100** serves as a peeler for cover **300,** optionally at least its protective liner layer. For example, needle cap remover **100** is engaged with extension **254** through orifice **305.** Optionally, user handle **190** which is optionally connected needle cap remover body **120,** does not pull cover **300** directly away from the whole surface of the device base all at once (an act that might require a large force to overcome the sticking force over a large surface). In some embodiments, when handle **190** at is pulled, extension **254** pulls with it section **256** followed by section **250.** In some embodiments, extension **304** guides the peeling initiation direction of section **250.** In some embodiments, folded section **256** may unfurl, unfold, stretch and/or bend to allow a certain distance to build up. Optionally, unfolding of section **256** together with peeling of extension **304,** converts the linear force away from housing **200** to a peeling force at the edge of adhesion of adhesive cover adhesive cover. The peeling force may optionally be along the surface of base of housing **200** and/or the peeling force may be directed away from the surface at an angle. For example, the angle of the peeling may range for example between 60-90 degrees and/or between 30-60 degrees and/or between 0 and 30 degrees.

*Fig. 3G* illustrates details of possible structure of cover **300**, having at least two active zones including an adhesive formed on a surface according to some embodiments of the current invention. In some embodiments a wall of a device (for example base of housing **200**) may have an active surface (for example adhesive **250a** and/or **250d**). Optionally the adhesive may be formed in layers. For example, as illustrated in *Fig. 3G*, adhesive portion of cover **300** includes three layers. A first layer **250a** includes a dual sided adhesive. Adhesive may for example, join a semi-stiff membrane **250b** to the base **212**. Membrane **250b** may extend beyond the edges of the base, optionally extending in extension **304**. Potentially, a semi-stiff extension beyond the edges of the base may in some embodiments make the injector adhere more strongly the skin of a user. In some embodiments, supporting membrane **250c** is provided.

In some embodiments, the adhesive cover removal force ranges between 10-150 gr/cm. The adhesive cover removal force may be lower than the bending inertia of the adhesive membrane **250a**. For example this may prevent bending of membrane **250b** when removing the cover. An external face of membrane may optionally include an active surface. An active surface for the purpose of this application may be a surface that has active modality wherein the surface interacts with an external element facilitating the functioning of the device and an inactive modality wherein it substantially does not interact with the external element. For example, prior to enablement of the injector adhesive **250d** may be inactive and/or protected by adhesive cover **250e**.

Optionally, sections **250**, **254** and/or **252** have different numbers of layers. In some embodiments, section **254** comprises adhesive layer **252a** connecting base portion **214** to liner **252c** and its supporting layer **252b.** In some embodiments, extension **254** comprises extension of section **250** in the form of layer **254a**, and further comprises adhesive layer **254b** connecting to supporting layer **254c**.

### 4 Exemplary bifurcated needle cap remover

Reference is now made to *FIG. 4* illustrating various views of needle cap cover body **120**, in accordance with some embodiments of the invention. In some embodiments, cover body **120** is provided as a cylinder having a bore **124**. In some embodiments, a distal portion of body **120** is bifurcated. Optionally, at least two slits **101** provide the bifurcated portions, optionally creating at least two distinct portions.

In some embodiments, at least 4 slits **101** are provided such that at least 4 distinct portions are available at the distal end of body **120**. Optionally, at least two of the portions comprise elastic portions **130**. Alternatively or additionally, at least two of the portions comprise rigid portions **150**. Optionally, at least two elastic portions **130** are positioned substantially diametrically to one another and/or optionally at least two rigid portions **150** are positioned substantially diametrically to one another.

In some embodiments, elastic portions **130** have a length extending beyond a top sill of a needle cap, for example, beyond 0.5 mm, 1mm, or beyond 2 mm, or beyond 3 mm, or any length smaller, larger or intermediate. Optionally, elastic portions **130** extend in a range having length of about 3 mm to about 10 mm beyond the top rim of bore **124**. In some embodiments, extended elastic portions comprise snaps **132**, configured to overhang a top sill of needle cap **202.** In some embodiments, snaps **132** comprise projections, optionally extending into an inner radial direction of bore **124**. Optionally, the projections have a length of about 0.2 mm. Alternatively, the projections have a length of about 0.1 mm. the projections have a length of about 0.3 mm, or any length smaller, larger or intermediate to the mentioned lengths herein.

In some embodiments, intermediate slits **104**, optionally not extending all the way to the distal rim of bore **124**, are provided, optionally in elastic portions **130.** Potentially, a configuration of positioning slits **101** and/or **104**, and/or their size and/or shapes affect an elastic extent of elastic portions **130** and a rigidness extent of rigid portions **150.** In some embodiments, the configuration and/or shape and/or size of slits **101** and/or **104** are configured to allow a small amount of force to push body **120** onto the needle cap and a large amount of force to pull body **120** away from the needle cap, optionally resulting in removal of the needle cap together with pulling of body **120.** For example, a small amount of force is in the range of about 50 g to about 400 g. Alternatively, small force is in the range of about 100 g to about 300 g. Alternatively, small force is in the range of about 100g to about 150 g. Optionally, a large amount of force is in the range of about 0.5-1.5 Kg. Alternatively, large amount of force is in the range of about 0.75 Kg to about 1.25 Kg. Alternatively, large amount of force is no more than 1 Kg.

In some embodiments, body **120** comprises at least one fastener **410**, optionally having fastener lock **412** such as for example a projection, optionally located at the proximal portion of body **120.** Potentially, fastener **410** is configured to snap and/or lock with complementary portions in connector body **170**, potentially stabilizing body **120** in place. Alternatively or additionally, connector body **170** connects body **120** with handle **190**. A potential advantage of providing multiple parts is to enable more degrees of freedom in an assembly process. In some embodiments, at least 3 fasteners **410** are provided, optionally equidistant from each other.

Reference is now made to *FIGs. 4E and 4F*, illustrating another embodiment of a needle cap remover, in accordance with some embodiments of the invention, and having at least four fasteners **410**. *FIG. 4E* illustrates a front view of body cover **120** and *FIG. 4F* illustrates a front view of body **120** when connected to connector body **170** and while enveloping cap **202**, which shields a needle connected to cannula **204** which is coupled to injector **206**. Fasteners **410** are optionally coupled in their position to the position of the bifurcations. In some embodiments, fasteners **410** are provided in the rigid portions **150** of cover body **120**. Optionally, cover body **120** comprises crack **430**, possibly in the form of a non-straight line. A potential advantage of crack **430** is allowing more flexibility. A potential advantage of a non-straight form is allowing more stability.

Reference is now made to *FIGs. 4G and 4H*, illustrating a cross section front view of the cap remover being pushed over the needle cap, in accordance with some embodiments of the invention, wherein *FIG. 4G* illustrates the needle cap remover being pushed mid-way along the needle cap and *FIG. 4H* illustrates the needle cap remover after snapping into place and having snaps **132** overhanging the top sill of the needle cap.

In some embodiments, the perimeter of the cap is not uniform, and it optionally includes a larger perimeter at its top end, i.e. the location where the needle projects outward from the syringe body. In some embodiments, hooks **132** are configured to define a perimeter which in its relaxed, unstressed state, can contains the perimeter of the bottom portion of cap **202**, but cannot contain the perimeter of the top portion of cap **202**. In some embodiments, the top portion of cap **202** comprises depressions for clasping hooks **132**.

Reference is now made to *FIG. 4I*, illustrating a perspective partial view of cartridge **206**, having a needle cap **202** protecting a needle connected to cannula **204**, assembled with a cap remover device in accordance with some embodiments of the invention. In some embodiments, hooks **132** are configured to deflect elastic portions **130** away from the central axis of needle cap **202,** optionally when passing by perimeters which are larger than defined by hooks **132.** In some embodiments, hooks **132** are configured to overhang a top sill **402** of cap **202,** optionally once the hooks pass the top portion of cap **202**.

In some embodiments, hooks **132** include projections in a direction towards a central axis of the remover device. Optionally, the projections **132** are perpendicular to the longitudinal axis of the cover body **120**. Alternatively, the projections are tilted towards the proximal portion of cover body **120**. In some embodiments, the tilting of the projections **132** affects their resistance when pulling the cover body **120** with respect to cap **202**. Optionally, a pulling force over cover body **120** causes projections **132** to increase their tilting angle with respect to the longitudinal axis of cover **120,** potentially causing a mechanical interference resisting the pulling of cover body **120** without also pulling needle cap **202**.

### 5 An exemplary connector for a needle cap remover

Reference is now made to *FIG. 5*, illustrating a connector in the form of connector **170**, in accordance with some embodiments of the current invention. In some embodiments, connector body **170** comprises a bore **574** sized and shaped to fit a needle cap remover cover body such as cover body **120,** and/or to fit the needle cap proximal portion. In some embodiments, connector body **170** is provided with at least one complementary portion configured to lock connector body **170** onto cover body **120**, optionally through fastener **410**. In some embodiments, a connector undercut **578** is provided, enveloping at least a portion of connector bore **574** and optionally configured to clasp onto at least one fastener **410**. In some embodiments, fastener **410** is shaped to allow easy insertion of connector body **170** in the direction towards the distal portion of cover body **120**, but optionally clamp the connector **170** to the cover body **120** once undercut **578** has passed the fasteners position.In some embodiments, fasteners **410** are configured to prevent a lateral movement of the needle cap remover cover body with respect to the needle cap.

In some embodiments, handle **190** is connected to connector body **170**, optionally through connector **572.** Alternatively or additionally, handle **190** is made as a single unit together with connector body **170** and/or connector base **174.**

### 6 Assembling a needle cap remover onto a needle cap

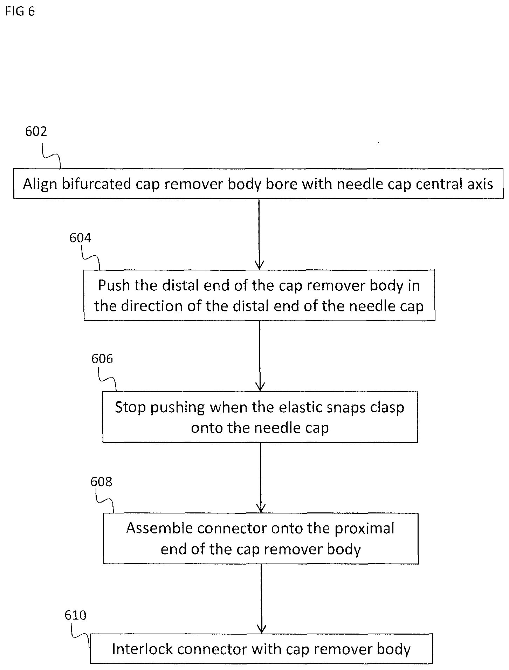

Reference is now made to *FIG.* 6, having a flow chart illustrating an exemplary assembly of needle cap remover onto a needle cap, in accordance with some embodiments of the invention. In some embodiments, a cap remover body is aligned with a needle cap central axis **602**. Alignment potentially prevents an excessive force exertion when pushing the remover onto the cap, which could lead to harming the needle, and/or disturbing the sterility of the inner zone of the needle cap.

In some embodiments, once the cap remover is substantially aligned with the needle cap, the cap remover is pushed towards the needle cap **604** optionally by inserting the proximal end of the needle cap into the distal end of the cap remover and pushing the distal end of the cap remover towards the direction of the distal end of the needle cap. Having bifurcations in the cap remover body potentially leads to elastic portions in the cap remover. In some embodiments, once the cover is pushed, the elastic portions, comprising snaps which optionally define a smaller circumference than the circumference of the needle cap, are pushed away from the central longitudinal axis of the cap remover.

In some embodiments, pushing of the cap remover stops when the elastic snaps reach beyond the top portion of the needle cap **606.** In some embodiments, an interlocking mechanism prevents pushing of the cap remover beyond a certain point above the needle cap, such as for example, fasteners **410**. Alternatively or additionally, the pushing of the cap remover stops when a sensor senses a reduction in the force resisting the push. In some embodiments, the snaps bounce into place thanks to the elastic properties of the elastic portions, optionally leading to the snaps overhanging a top sill of the needle cap.

Optionally, a connector, for example in the form of a clamp, is assembled onto the proximal end of the cap remover body **608**. The connector potentially holds the cap remover body still with respect to the housing of an injector, and/or allow connection of the cap remover to additional features, such as a user handle and/or a liner covering operation features, for example adhesive portions and/or battery insulators.

In some embodiments, connector is pushed onto remover body until it interlocks in place **610**, optionally by interlocking with at least one fastener **410** provided in the cap remover body.

Alternatively or additionally, the clamp, optionally already associated with a user handle, is interconnected to cap remover body prior to its enveloping onto the needle cap.

### 7 Assembling a needle cap remover having an adhesive liner, onto a needle cap

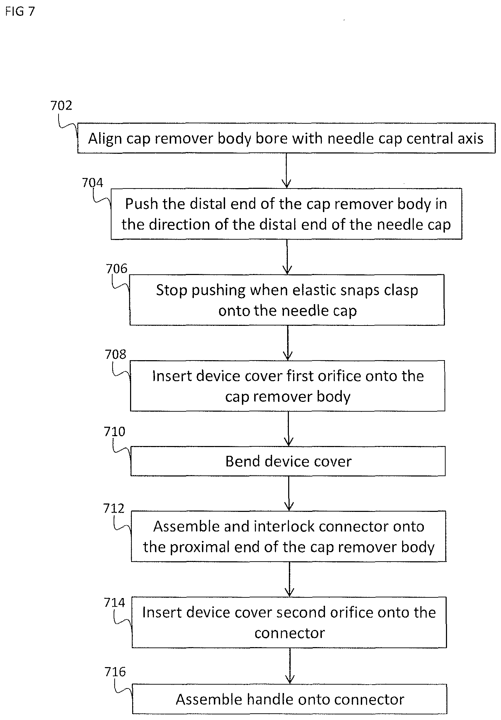

Reference is now made to *FIG.* 7, showing a flow chart illustrating an exemplary assembly process of a needle cap remover associated with a device cover, such as for example an adhesive liner, in accordance with some embodiments of the invention.

In some embodiments, aligning the central axis of the cap remover body with central axis of the needle cap **702** is done initially to prevent unnecessary force exertion. In some embodiments, the cap remover is then pushed onto the needle cap **704** as shown in **604**, and optionally pushing is stopped when the body is clasped onto the cap **706**, optionally by means of elastic snaps.

In some embodiments, the cap remover device is being inserted into a first orifice provided in a device cover **708**, optionally comprising an adhesive liner. In some embodiments, a connector is assembled onto the cap remover **712,** optionally securing the cover onto the cap remover.

In some embodiments, the device cover is bent **710,** optionally at a portion which extends beyond the border of the injection system, and the connector body is inserted into a second orifice provided in the cover **714.** Optionally, the second portion of the cover is secured after assembly of the user handle onto the connector **716.**

### 8 Exemplary assembly process of an automatic needle injector device

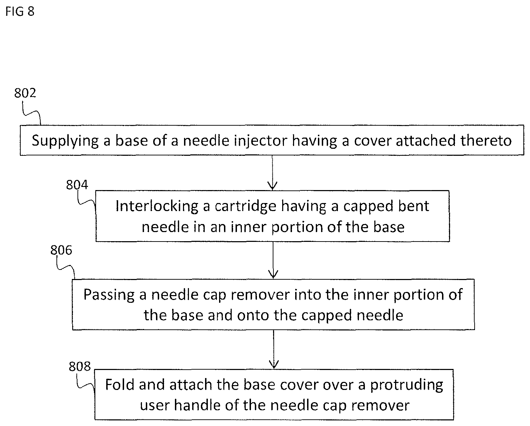

Reference is now made to *FIG. 8*, illustrating an exemplary process of an assembly process of an automatic needle injector device, in accordance with some embodiments of the invention.

In some embodiments, a base of a needle injector housing is supplied **802**, optionally being attached to a protective cover. In some embodiments, the protective cover comprises at least one adhesive layer, optionally for positioning and stabilizing the injector over a user's body. In some embodiments, the adhesive layer is protected by a second layer being a protective liner.

In some embodiments, a prefilled cartridge, optionally having a bend needle path, is positioned inside the base and interlocked to be engaged with it **804**. Optionally, the cartridge is positioned such that a central axis of the cartridge to be parallel to the base-cover. In some embodiments, the cartridge comprises a capped bent needle. Optionally, the bent needle is centrally aligned with an orifice provided within the base, potentially allowing access from the outside of the injector housing, to the needle optionally residing within the injector housing. In some embodiments, the device cover covering the base also comprises a first aperture, optionally matching to the orifice of the base.

In some embodiments, a needle cap remover is passed from the outside portion of the base into the inside portion of the base, through the first aperture of the device cover **806**. Optionally, the needle cap remover is pushed onto the capped needle. In some embodiments, the first aperture is sized and shaped to fit at least the circumference of the needle cap remover. In some embodiments, the needle cap remover is placed onto the capped needle in its distal end, optionally being in the inner portion of the injector. Optionally, the needle cap remover comprises in its proximal end a user handle, potentially being used to remover the needle cap remover with the needle cap.

In some embodiments, the needle cap remover is placed onto the capped needle while being attached to the user handle. Optionally, a cover edge which is not attached to the outer portion of the base, is folded over and attached to the user handle **808**. In some embodiments, when pulling the user handle linearly, the cover edge is pulled with the handle and its connected needle cap remover, while the cover portion attached to the device is peeled from the direction of the edge.

In some embodiments, the user handle comprises a protruding member having a short dimension and a long dimension. Optionally, the cover is attached to the user handle by passing a second aperture of the cover over the protruding portion of the user handle. In some embodiments, the second aperture is assembled onto the user handle by orienting the second aperture of the cover to face the short dimension of the handle and rotating the cover onto the protruding element until the second aperture is oriented to face the long dimension.

### 9 Exemplary multi-part cap remover

In some embodiments, a cap remover is provided by having multiple parts. Optionally, the parts are assembled to a complete cap remover only while and/or after assembling onto the needle cap.

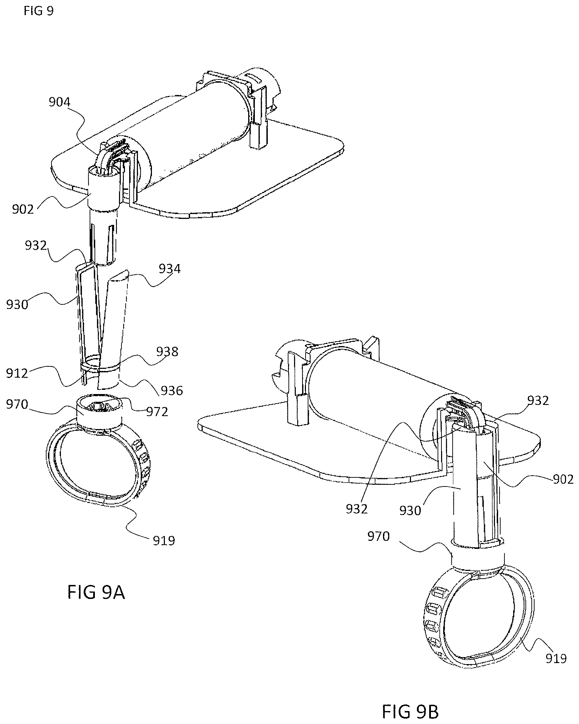

Reference is now made to *FIGs. 9A-B*, illustrating an exemplary embodiment of a multi part cap remover in accordance with some embodiments of the current invention. In some embodiments, a needle cap remover is provided having at least two elongated arms **930**, sized and shaped to fit a longitudinal portion of a needle cap wall **902**, optionally serving to protect cannula **904.** Optionally, a distal portion **934** of the arms comprise enclosing members **932,** optionally sized and shaped to fit a top surface of a needle cap. In some embodiments, a connector body **970** is sized and shaped to enclose over the at least two arms **930**, optionally at their proximal portion **936,** optionally leading to their clasping over the cap.

In some embodiments, an intermediate portion **938** of arm **930** comprises at least one fastening member; such as for example ring **912**. Optionally, member **912** is configured to interlock with fastener **972** provided within connector body **970**.

*FIG. 9A* illustrates an exemplary open configuration of the cap remover, having arms **930** tilted away from the longitudinal axis in their distal portion **934**, optionally while being held together by ring **912**. *FIG. 9B* illustrates an exemplary closed configuration, illustrating the enveloping of arms **930** around the needle cap, and their optional securing mechanism provided by connector **970**, which optionally further comprises a user handle **919**.

### 10 Assembling a multi-part cap remover onto a needle cap

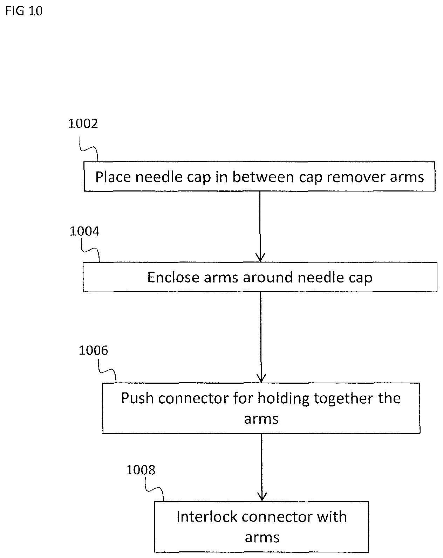

Reference is now made to *FIG. 10*, showing a flow chart illustrating an exemplary assembly process of a multi-part needle cap remover, in accordance with some embodiments of the current invention.

In some embodiments, a needle cap is placed in between the arms of a multi-part cap remover **1002**. A potential advantage of having multiple parts is a flexibility in the perimeter defined by the arms. Optionally when the arms are open, they define a perimeter which is larger than the perimeter of the needle cap, potentially allowing their placement with a relatively low amount of force and/or low precision of aligning the central axis of the cap with the central axis of the cap remover.

Optionally, the arms enclose over the cap **1004** when a securing connector is pushed **1006** onto the proximal end of the arms, tilting them towards the needle cap. In some embodiments, the needle cap remover is held securely over the needle cap once the securing connector interlocks with the arms **1008** leading to securement of the arms over the needle cap. In some embodiments, the connector interlocks with the arms by clasping over the coupling element which mechanically couples the two arms, optionally the coupling element being a ring.

In some embodiments, no more than 25 g of force is exerted in order to interlock the connector with the arms, leading to their closed configuration. Alternatively, no more than 50 g force is exerted. Alternatively, no more than 100 g force is exerted. In some embodiments, in order to pull the needle cap no more than 0.5 Kg force is applied. Alternatively, no more than 0.7 Kg force is applied. Alternatively, no more than 0.9 Kg force is applied. Alternatively, no more than 1 Kg force is applied. Alternatively, no more than 1.2 Kg force is applied. Alternatively, no more than 1.5 Kg force is applied.

### 11 Exemplary of a needle cap remover having a single arm

In some embodiments, a cover body comprising a single arm **1120** is provided. Optionally, arm **1120** comprises a tubular shape. In some embodiments, slit **1101** is provided along the central longitudinal axis of arm **1120,** optionally extending along the entire length of arm **1120**. Potentially, slit **1101** provides elasticity to arm **1120**, enabling the width of **1120** to open when force is exerted. In some embodiments, undercut **1132** is provided in at least a portion of the inner circumference of the top sill of arm **1120**. Undercut **1132** is optionally sized and shaped to allow relatively easy insertion against a surface, together with relatively difficult removal, possibly due to its snap enablement. In some embodiments, aperture **1133** is provided to allow room for features comprised in the injector, which may hinder the space available for arm **1120**.

As used herein the term "about" refers to ± 25 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A device (100) for removing a needle cap (202), the needle cap (202) shielding a needle (302) of an injector system, the needle (302) being located at a proximal side of the injector system, said device comprising:
an elongated hollow body (120) that is cylindrical, having a distal end (B) sized and shaped to at least partially envelop the needle cap (202), and at least three fasteners (410) located at a proximal portion of the body (120) equidistant from each other;
at least two elastic arms (130) defined as a surface between two slits (101), said two slits (101) extending from the distal end (B) of said elongated hollow body (120); at least two snaps (132) coupled to the distal ends of the at least two elastic arms (130);
a user handle (190) having a protruding element; and
a connector body (170) connecting the body (120) with the user handle (190), the connector body (170) having a connector bore (574) sized and shaped to fit the proximal portion of said body (120), wherein said fasteners (410) are configured to snap into complementary portions in the connector body (170) to stabilize the body (120),
said body (120) having a closed and open configuration, wherein the closed configuration is defined by the at least two snaps (132) defining a width smaller than a width of a top sill (402) of the needle cap (202), and said open configuration is defined by the at least two snaps (132) elastically deflecting away from a central axis (602) of the needle cap (202), and wherein once said at least two snaps (132) are pushed beyond the top sill (402) of the needle cap (202), the at least two elastic arms (130) return to the closed configuration and the at least two snaps (132) overhang the top sill (402) of the needle cap (202) with said body (120) enveloping a lower portion of said needle cap (202).

2. The device (100) of claim 1, wherein said at least two snaps (132) inflect towards the central axis (602) of the needle cap (202), being angled with respect to said central axis (602).

3. The device (100) of claim 1, further comprising at least one intermediate longitudinal slit (104) provided in said surface between said two slits (101).

4. The device (100) of any of claims 1-3, wherein said body (120) further comprises at least two guides along its inner surface and oriented along a longitudinal axis of said body (120), said at least two guides sized and shaped to accommodate complementary elements positioned on an outer surface of said needle cap (202).

5. The device (100) of claim 1, wherein said body (120) further comprises at least one interlocking element complementary to an interlocking member (410) positioned in said connector body (170), thereby inhibiting a lateral movement of said body (120) with respect to said connector.

6. A system for medicament delivery, comprising:
a cartridge (206) containing a medicament and being in fluid communication with a needle, said needle enveloped by the needle cap (202);
a housing (200) for containing said cartridge (206) and having an orifice for allowing access to said needle cap (202) and its associated needle; and
the device (100) of any preceding claim.

7. The system of claim 6, further comprising an adhesive layer having a protective liner, said adhesive layer connected to said housing (200), wherein said protective liner extends beyond a surface of said housing (200) and positioned between said body (120) and a connector.

8. A method of assembling a needle cap remover (100) according to any of claims 1-5 onto the needle cap (202), comprising:
aligning a longitudinal axis of the body (120) to be colinear with a longitudinal axis (602) of the needle cap (202); and
inserting the distal end (B) of the body (120) in the direction of the top sill (402) of the needle cap (202) while deflecting the at least two elastic arms (130) of said body (120) in a direction away from the central axis (602) of the needle cap (202), said deflecting is provided by the at least two snaps (132) defining a smaller perimeter than a perimeter of the needle cap (202),
wherein said inserting is provided until the at least two snaps (132) extend beyond the top sill (402) of the needle cap (202) and snap inwardly towards the central axis (602) of the needle cap (202), thereby overhanging the top sill (402) of said needle cap (202), causing the at least two elastic arms (130) to snap from said deflecting towards the central axis (602) of the needle cap (202).

9. The method according to claim 8, wherein the needle cap (202) is comprised within an injector system and said method further comprises inserting the needle cap remover (100) through an orifice in the injector system having access to the needle cap (202).

10. The method according to any of claims 8-9, further comprising interlocking a proximal portion of said body (120) with the connector body (170) having the user handle (190).

## Patentansprüche

1. Vorrichtung (100) zum Entfernen einer Nadelkappe (202), wobei die Nadelkappe (202) eine Nadel (302) eines Injektorsystems abschirmt, wobei die Nadel (302) an einer proximalen Seite des Injektorsystems gelegen ist, wobei die genannte Vorrichtung umfasst:
einen länglichen Hohlkörper (120), der zylindrisch ist, der ein distales Ende (B), das so bemessen und geformt ist, dass es zumindest teilweise die Nadelkappe (202) umhüllt, und zumindest drei Verschlüsse (410) aufweist, die an einem proximalen Abschnitt des Körpers (120), gleich voneinander entfernt, gelegen sind;
zumindest zwei elastische Arme (130), die als eine Fläche zwischen zwei Schlitzen (101) definiert sind, wobei die genannten zwei Schlitze (101) sich von dem distalen Ende (B) des genannten länglichen Hohlkörpers (120) erstrecken;
zumindest zwei Schnappschlösse (132), die an den distalen Enden der zumindest zwei elastischen Arme (130) gekoppelt sind;
einen Benutzergriff (190), der ein vorstehendes Element aufweist; und
einen Verbinderkörper (170), der den Körper (120) mit dem Benutzergriff (190) verbindet, wobei der Verbinderkörper (170) eine Verbinderbohrung (574) hat, die so bemessen und geformt ist, dass sie zum proximalen Abschnitt des Körpers (120) passt, worin die Verschlüsse (410) dafür ausgelegt sind, um in komplementäre Abschnitte im Verbinderkörper (170) einzuschnappen, um den Körper (120) zu stabilisieren,
der Körper (120) eine geschlossene und eine offene Konfiguration aufweist, worin die geschlossene Konfiguration von den zumindest zwei Schnappschlössen (132) definiert ist, welche eine Breite kleiner als eine Breite eines oberen Randes (402) der Nadelkappe (202) definieren, und die offene Konfiguration von den zumindest zwei Schnappschlössen (132) definiert ist, welche elastisch weg von einer Mittelachse (602) der Nadelkappe (202) ablenken, und worin, nachdem die genannten zumindest zwei Schnappschlösse (132) über den oberen Rand (402) der Nadelkappe (202) hinaus geschoben werden, die zumindest zwei elastischen Arme (130) zur geschlossenen Konfiguration zurückgehen und die zumindest zwei Schnappschlösse (132) über den oberen Rand (402) der Nadelkappe (202) mit dem Körper (120), der einen unteren Abschnitt der Nadelkappe (202) umhüllt, auskragen.

2. Vorrichtung (100) nach Anspruch 1, worin die genannten zumindest zwei Schnappschlösse (132) sich zur Mittelachse (602) der Nadelkappe (202) biegen, wobei sie im Hinblick auf die Mittelachse (602) abgewinkelt sind.

3. Vorrichtung (100) nach Anspruch 1, ferner umfassend zumindest einen zwischenliegenden Längsschlitz (104), der in der Fläche zwischen den zwei Schlitzen (101) vorgesehen ist.

4. Vorrichtung (100) nach einem der Ansprüche 1-3, worin der Körper (120) ferner zumindest zwei Führungen entlang seiner inneren Fläche und entlang einer Längsachse des Körpers (120) ausgerichtet umfasst, wobei die genannten zumindest zwei Führungen so bemessen und geformt sind, dass sie komplementäre Elemente beherbergen, die an einer äußeren Fläche der Nadelkappe (202) positioniert sind.

5. Vorrichtung (100) nach Anspruch 1, worin der Körper (120) ferner zumindest ein Verriegelungselement umfasst, das zu einem Verriegelungsglied (410) komplementär ist, das im Verbinderkörper (170) positioniert ist, wodurch eine seitliche Bewegung des Körpers (120) im Hinblick auf den Verbinder verhindert wird.

6. System zur Medikamentenabgabe, umfassend:
eine Kartusche (206), die ein Medikament umfasst und in Fluidkommunikation mit einer Nadel steht, wobei die Nadel von der Nadelkappe (202) umhüllt ist;
ein Gehäuse (200) zum Enthalten der Kartusche (206), und das eine Öffnung hat, um Zugang zur Nadelkappe (202) und zu ihrer dazugehörigen Nadel zu ermöglichen; und
die Vorrichtung (100) nach einem der vorhergehenden Ansprüche.

7. System nach Anspruch 6, ferner umfassend eine Klebeschicht, die eine Schutzauskleidung hat, wobei die Klebeschicht mit dem Gehäuse (200) verbunden ist, worin die Schutzauskleidung sich über eine Fläche des Gehäuses (200) hinaus erstreckt und zwischen dem Körper (120) und einem Verbinder positioniert ist.

8. Verfahren zur Montage eines Nadelkappenentferners (100) nach einem der Ansprüche 1-5 an der Nadelkappe (202), umfassend:
Ausrichten einer Längsachse des Körpers (120), damit sie zu einer Längsachse (602) der Nadelkappe (202) kollinear ist; und
Einfügen des distalen Endes (B) des Körpers (120) in die Richtung des oberen Randes (402) der Nadelkappe (202), während die zumindest zwei elastischen Arme (130) des Körpers (120) in eine Richtung weg von einer Mittelachse (602) der Nadelkappe (202) abgelenkt werden, wobei das Ablenken von den zumindest zwei Schnappschlössen (132) bereitgestellt wird, welche einen kleineren Umfang als den Umfang der Nadelkappe (202) definieren,
worin das Einfügen vorgesehen ist, bis die zumindest zwei Schnappschlösse (132) sich über den oberen Rand (402) der Nadelkappe (202) hinaus erstrecken und nach innen zur Mittelachse (602) der Nadelkappe (202) einschnappen, wodurch sie über den oberen Rand (402) der Nadelkappe (202) auskragen, was bewirkt, dass die zumindest zwei elastischen Arme (130) von dem Ablenken zur Mittelachse (602) der Nadelkappe (202) einschnappen.

9. Verfahren nach Anspruch 8, worin die Nadelkappe (202) innerhalb eines Injektorsystem umfasst ist und das Verfahren ferner das Einfügen des Nadelkappenentferners (100) durch eine Öffnung im Injektorsystem, die zur Nadelkappe (202) Zugang hat, umfasst.

10. Verfahren nach einem der Ansprüche 8-9, ferner umfassend das Verriegeln eines proximalen Abschnitts des Körpers (120) mit dem Verbinderkörper (170), der den Benutzergriff (190) aufweist.

## Revendications

1. Dispositif (100) pour retirer un capuchon (202) d'aiguille, le capuchon (202) d'aiguille protégeant une aiguille (302) d'un système d'injection, l'aiguille (302) étant située sur un côté proximal du système d'injection, ledit dispositif comprenant :
un corps creux allongé (120) qui est cylindrique, présentant une extrémité distale (B) dimensionnée et formée pour envelopper au moins partiellement le capuchon (202) d'aiguille, et au moins trois éléments de fixation (410) situés au niveau d'une partie proximale du corps (120), équidistants les uns des autres ;
au moins deux bras élastiques (130) définis en tant que surface entre deux fentes (101), lesdites deux fentes (101) s'étendant à partir de l'extrémité distale (B) dudit corps creux allongé (120) ;
au moins deux éléments encliquetables (132) couplés aux extrémités distales des au moins deux bras élastiques (130) ;
une poignée (190) d'utilisateur présentant un élément saillant ; et
un corps de raccordement (170) reliant le corps (120) à la poignée (190) d'utilisateur, le corps de raccordement (170) présentant un alésage de raccordement (574) dimensionné et formé pour s'adapter à la partie proximale dudit corps (120), dans lequel lesdits éléments de fixation (410) sont configurés pour s'encliqueter dans des parties complémentaires dans le corps de raccordement (170) pour stabiliser le corps (120),
ledit corps (120) présentant une configuration fermée et ouverte, dans lequel la configuration fermée est définie par les au moins deux éléments encliquetables (132) définissant une largeur inférieure à une largeur d'un rebord supérieur (402) du capuchon (202) d'aiguille, et ladite configuration ouverte est définie par les au moins deux éléments encliquetables (132) s'écartant élastiquement d'un axe central (602) du capuchon (202) d'aiguille, et dans lequel une fois lesdits au moins deux éléments encliquetables (132) poussés au-delà du rebord supérieur (402) du capuchon (202) d'aiguille, les au moins deux bras élastiques (130) reviennent à la configuration fermée et les au moins deux éléments encliquetables (132) surplombent le rebord supérieur (402) du capuchon (202) d'aiguille avec ledit corps (120) enveloppant une partie inférieure dudit capuchon (202) d'aiguille.

2. Dispositif (100) selon la revendication 1, dans lequel lesdits au moins deux éléments encliquetables (132) s'infléchissent vers l'axe central (602) du capuchon (202) d'aiguille, étant inclinés par rapport audit axe central (602).

3. Dispositif (100) selon la revendication 1, comprenant en outre au moins une fente longitudinale intermédiaire (104) ménagée dans ladite surface entre lesdites deux fentes (101).

4. Dispositif (100) selon l'une quelconque des revendications 1-3, dans lequel ledit corps (120) comprend en outre au moins deux guides le long de sa surface intérieure et orientés le long d'un axe longitudinal dudit corps (120), lesdits au moins deux guides étant dimensionnés et formés pour recevoir des éléments complémentaires positionnés sur une surface extérieure dudit capuchon (202) d'aiguille.

5. Dispositif (100) selon la revendication 1, dans lequel ledit corps (120) comprend en outre au moins un élément d'enclenchement complémentaire d'un organe d'enclenchement (410) positionné dans ledit corps de raccordement (170), empêchant ainsi un mouvement latéral dudit corps (120) par rapport audit raccord.

6. Système de distribution de médicament, comprenant :
une cartouche (206) contenant un médicament et étant en communication fluidique avec une aiguille, ladite aiguille étant enveloppée par le capuchon (202) d'aiguille ;
un boîtier (200) destiné à contenir ladite cartouche (206) et présentant un orifice pour permettre l'accès audit capuchon (202) d'aiguille et à son aiguille associée ; et
le dispositif (100) selon une quelconque revendication précédente.

7. Système selon la revendication 6, comprenant en outre une couche adhésive présentant un revêtement protecteur, ladite couche adhésive étant reliée audit boîtier (200), dans lequel ledit revêtement protecteur s'étend au-delà d'une surface dudit boîtier (200) et est positionné entre ledit corps (120) et un raccord.

8. Procédé d'assemblage d'un dispositif de retrait (100) de capuchon d'aiguille selon l'une quelconque des revendications 1-5 sur le capuchon (202) d'aiguille, comprenant :
l'alignement d'un axe longitudinal du corps (120) pour qu'il soit colinéaire avec un axe longitudinal (602) du capuchon (202) d'aiguille ; et
l'insertion de l'extrémité distale (B) du corps (120) en direction du rebord supérieur (402) du capuchon (202) d'aiguille tout en faisant dévier les au moins deux bras élastiques (130) dudit corps (120) dans une direction s'éloignant de l'axe central (602) du capuchon (202) d'aiguille, ladite déviation est assurée par les au moins deux éléments encliquetables (132) définissant un périmètre plus petit qu'un périmètre du capuchon (202) d'aiguille,
dans lequel ladite insertion a lieu jusqu'à ce que les au moins deux éléments encliquetables (132) s'étendent au-delà du rebord supérieur (402) du capuchon (202) d'aiguille et s'encliquettent vers l'intérieur vers l'axe central (602) du capuchon (202) d'aiguille, surplombant ainsi le rebord supérieur (402) dudit capuchon (202) d'aiguille, provoquant l'encliquetage des au moins deux bras élastiques (130) passant de ladite déviation à l'axe central (602) du capuchon (202) d'aiguille.

9. Procédé selon la revendication 8, dans lequel le capuchon (202) d'aiguille est compris dans un système d'injection et ledit procédé comprend en outre l'insertion du dispositif de retrait (100) de capuchon d'aiguille à travers un orifice dans le système d'injection ayant accès au capuchon (202) d'aiguille.

10. Procédé selon l'une quelconque des revendications 8-9, comprenant en outre l'enclenchement d'une partie proximale dudit corps (120) avec le corps de raccordement (170) présentant la poignée (190) d'utilisateur.
